(19) Europäisches Patentamt
European Patent Office
Office européen des brevets

(11) Veröffentlichungsnummer: **0 368 158**
**A1**

# (12) EUROPÄISCHE PATENTANMELDUNG

(21) Anmeldenummer: **89120355.6**

(22) Anmeldetag: **03.11.89**

(51) Int. Cl.$^{5}$: **C07D 471/04, A61K 31/435, //(C07D471/04,235:00,221:00)**

(30) Priorität: **07.11.88 CH 4120/88**

(43) Veröffentlichungstag der Anmeldung:
**16.05.90 Patentblatt 90/20**

(84) Benannte Vertragsstaaten:
**ES GR**

(71) Anmelder: **Byk Gulden Lomberg Chemische Fabrik GmbH**
**Byk-Gulden-Strasse 2**
**D-7750 Konstanz(DE)**

(72) Erfinder: **Rainer, Georg, Dr.**
**Birnauerstrasse 23**
**D-7750 Konstanz(DE)**
Erfinder: **Schaefer, Hartmann, Dr.**
**Oberdorfstrasse 4**
**D-7750 Konstanz 18(DE)**
Erfinder: **Senn-Bilfinger, Jörg, Dr.**
**Säntisstrasse 7**
**D-7750 Konstanz(DE)**
Erfinder: **Grundler, Gerhard, Dr.**
**Meersburgerstrasse 4**
**D-7750 Konstanz(DE)**
Erfinder: **Klemm, Kurt, Prof. Dr.**
**Im Weinberg 2**
**D-7753 Allensbach(DE)**
Erfinder: **Simon, Wolfgang-Alexander, Dr.**
**Seestrasse 31a**
**D-7750 Konstanz(DE)**
Erfinder: **Riedel, Richard, Dr.**
**Durlesbach 7**
**D-7967 Bad Waldsee-Reute(DE)**
Erfinder: **Postius, Stefan, Dr.**
**Austrasse 4b**
**D-7750 Konstanz(DE)**

## (54) Neue Imidazopyridine.

(57) Imidazopyridine der Formel I,

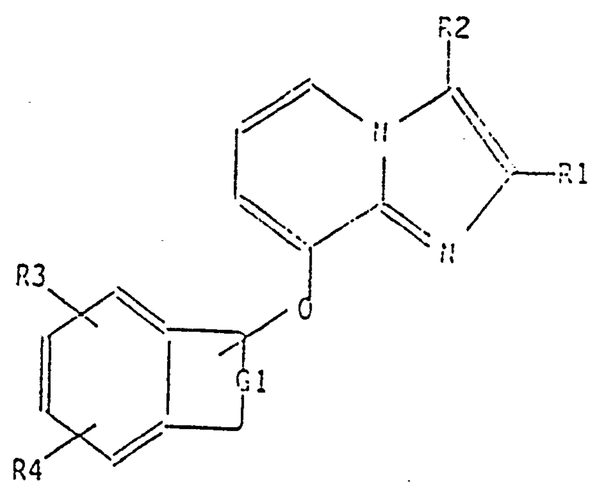

worin
R1 H, Alkyl, Hydroxyalkyl, Haloalkyl, Cyanoalkyl oder Alkoxycarbonyl,

EP 0 368 158 A1

R2 H, Alkyl, Formyl, Hydroxyalkyl, Haloalkyl, Cyanoalkyl oder Dialkylaminoalkyl,
R3 H, Alkoxy oder Halogen,
R4 H, Alkoxy oder Halogen und
G1 eine substituierte Gruppe der Formel $-CH(OR5)-CH_2-CH_2-$, $-CH_2-CH(OR5)-CH(CH_3)-$, $-CH_2-CH-(OR5)-CH_2-$, $-CH(OR5)-CH_2-CH_2-CH_2-$, $-CH_2-CH(OR5)-CH_2-CH_2-$, $-CH(OR5)-CH_2-CH_2-CH_2-CH_2-$ oder $-CH_2-CH(OR5)-CH_2-CH_2-CH_2-$ bedeutet in der
R5 Alkyl, Cycloalkyl, Alkoxyalkyl, Arylalkyl, Alkylcarbonyl, Cycloalkylcarbonyl, Arylalkylcarbonyl, Alkoxyalkylcarbonyl, Alkoxyalkoxyalkylcarbonyl, Alkoxycarbonyl, Alkoxyalkoxycarbonyl, Arylcarbonyl, Furoyl oder eine Carbamoylgruppe der Formel

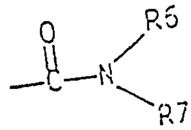

bedeutet, worin
R6 H, Alkyl, Hydroxyalkyl oder gemeinsam mit R7 und unter Einschluß des Stickstoffatoms, an das beide gebunden sind, Pyrrolidino, Piperidino, Tetrahydroazepino, Piperazino, N-Methylpiperazino oder Morpholino bedeutet und
R7 H, Alkyl, Aryl oder gemeinsam mit R6 und unter Einschluß des Stickstoffatoms, an das beide gebunden sind, Pyrrolidino, Piperidino, Tetrahydroazepino, Piperazino, N-Methylpiperazino oder Morpholino bedeutet, wobei
Aryl Phenyl oder substituiertes Phenyl mit einem, zwei oder drei Substituenten aus der Gruppe Halogen, Alkyl oder Alkoxy bedeutet,
sind neue Verbindungen. Sie weisen eine Magen- und Darmschutzwirkung bei Warmblütern auf.

# Neue Imidazopyridine

## Anwendungsgebiet der Erfindung

Die Erfindung betrifft neue Imidazopyridine, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

## Bekannter technischer Hintergrund

Aus den veröffentlichten Europäischen Patentanmeldungen 0 033 094, 0 068 378, 0 165 545 und 0 204 285 sind Imidazopyridine bzw. Imidazoisochinoline bekannt, die aufgrund ihrer antisekretorischen und cytoprotektiven Wirkung bei der Behandlung von Ulcuserkrankungen eingesetzt werden sollen.

## Beschreibung der Erfindung

Es wurde nun gefunden, daß die unten näher beschriebenen neuen Imidazopyridine interessante pharmakologische Eigenschaften aufweisen, durch die sie sich von den obenerwähnten bekannten Verbindungen in überraschender und besonders vorteilhafter Weise unterscheiden.

Gegenstand der Erfindung sind neue Imidazopyridine der Formel I

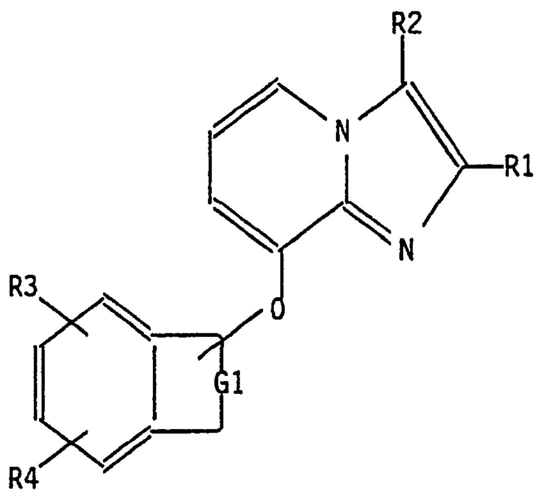

(I)

worin

R1 Wasserstoff (H), 1-4C-Alkyl, Hydroxy-1-4C-alkyl, Halo-1-4C-alkyl, Cyano-1-4C-alkyl oder 1-4C-Alkoxycarbonyl,

R2 Wasserstoff (H), 1-4C-Alkyl, Formyl, Hydroxy-1-4C-alkyl, Halo-1-4C-alkyl, Cyano-1-4C-alkyl oder Di-1-4C-alkylamino-1-4C-alkyl,

R3 Wasserstoff (H), 1-4C-Alkoxy oder Halogen,

R4 Wasserstoff (H), 1-4C-Alkoxy oder Halogen und

G1 eine substituierte Gruppe der Formel $-CH(OR5)-CH_2-CH_2-$, $-CH_2-CH(OR5)-CH(CH_3)-$, $-CH_2-CH(OR5)-CH_2-$, $-CH(OR5)-CH_2-CH_2-CH_2-$, $-CH_2-CH(OR5)-CH_2-CH_2-$, $-CH(OR5)-CH_2-CH_2-CH_2-CH_2-$ oder $-CH_2-CH(OR5)-CH_2-CH_2-CH_2-$ bedeutet in der

R5 1-6C-Alkyl, 3-7C-Cycloalkyl, 1-4C-Alkoxy-1-4C-alkyl, Aryl-1-4C-alkyl, 1-6C-Alkylcarbonyl, 3-7C-Cycloalkylcarbonyl, Aryl-1-4C-alkylcarbonyl, 1-4C-Alkoxy-1-4C-alkylcarbonyl, 1-4C-Alkoxy-1-4C-alkoxy-1-4C-alkylcarbonyl, 1-4C-Alkoxycarbonyl, 1-4C-Alkoxy-1-4C-alkoxycarbonyl, Arylcarbonyl, Furoyl oder eine Carbamoylgruppe der Formel

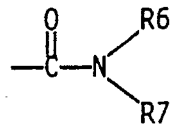

bedeutet, worin

R6 Wasserstoff (H), 1-4C-Alkyl, Hydroxy-1-4C-alkyl oder gemeinsam mit R7 und unter Einschluß des Stickstoffatoms, an das beide gebunden sind, Pyrrolidino, Piperidino, Tetrahydroazepino, Piperazino, N-Methylpiperazino oder Morpholino bedeutet und

R7 Wasserstoff (H), 1-4C-Alkyl, Aryl oder gemeinsam mit R6 und unter Einschluß des Stickstoffatoms, an das beide gebunden sind, Pyrrolidino, Piperidino, Tetrahydroazepino, Piperazino, N-Methylpiperazino oder Morpholino bedeutet, wobei

Aryl Phenyl oder substituiertes Phenyl mit einem, zwei oder drei Substituenten aus der Gruppe Halogen, 1-4C-Alkyl oder 1-4C-Alkoxy bedeutet,

und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest. Hydroxy-1-4C-alkyl steht für die vorstehend genannten 1-4C-Alkylreste, an die ein Hydroxylrest gebunden ist. Der bevorzugte Hydroxy-1-4C-alkylrest R2 ist der Hydroxymethylrest. Ein bevorzugter Hydroxy-1-4C-alkylrest R6 ist der 2-Hydroxyethylrest.

Halogen im Sinne der vorliegenden Erfindung ist Brom und insbesondere Chlor und Fluor.

Halo-1-4C-alkyl steht für die vorstehend genannten 1-4C-Alkylreste, an die ein Halogenatom gebunden ist. Bevorzugt ist der Chlormethylrest.

Cyano-1-4C-alkyl steht für die vorstehend genannten 1-4C-Alkylreste, an die ein Cyanrest gebunden ist. Bevorzugt ist der Cyanomethylrest.

Di-1-4C-alkylamino steht für Aminogruppen, die durch zwei der vorstehend genannten 1-4C-Alkylreste substituiert sind. Beispielsweise seien die Diisopropylamino- und insbesondere die Dimethylaminogruppe genannt. Di-1-4C-alkylamino-1-4C-alkyl steht für die vorstehend genannten 1-4C-Alkylreste, an die eine der vorstehend genannten Di-1-4C-alkylaminogruppen gebunden ist. Bevorzugt sind der Dimethylaminomethyl- und der Dimethylaminoethylrest.

1-4C-Alkoxyreste enthalten neben dem Sauerstoffatom einen der vorstehend genannten 1-4C-Alkylreste. Bevorzugt ist der Methoxyrest.

1-6C-Alkyl steht für geradkettige oder verzweigte Alkylreste mit 1 bis 6 Kohlenstoffatomen. Beispielsweise seien genannt der Hexyl-, Pentyl-, Butyl-, iso-Butyl-, sec.-Butyl-, tert.-Butyl-, Propyl-, Isopropyl-, Ethyl- und insbesondere der Methylrest.

3-7C-Cycloalkyl steht für den Cyclopropyl-, Cyclobutyl-, Cyclopentyl-, Cyclohexyl- und Cycloheptylrest.

1-4C-Alkoxy-1-4C-alkyl steht für 1-4C-Alkyl, das an beliebiger Position durch 1-4C-Alkoxy substituiert ist. Bevorzugt ist der Methoxyethylrest.

Aryl -1-4C-alkyl enthält an beliebiger Position im 1-4C-Alkyl rest einen Aryl-, bevorzugt einen Phenylrest. Bevorzugt ist der Benzylrest.

1-6C-Alkylcarbonyl enthält neben der Carbonylgruppe einen der vorstehend genannten 1-6C-Alkylreste. Bevorzugt ist der Methylcarbonyl-( = Acetyl-)rest.

3-7C-Cycloalkylcarbonyl enthält neben der Carbonylgruppe einen der vorstehend genannten 3-7C-Cycloalkylreste. Bevorzugt ist der Cyclopropylcarbonylrest.

Aryl-1-4C-alkylcarbonyl enthält an beliebiger Position im 1-4C-Alkylcarbonylrest einen Aryl-, bevorzugt einen Phenylrest. Bevorzugt ist der Benzylcarbonylrest.

1-4C-Alkoxy-1-4C-alkylcarbonyl enthält an beliebiger Position im 1-4C-Alkylcarbonylrest einen 1-4C-Alkoxy-, bevorzugt einen Methoxyrest. Bevorzugt sind der Methoxyacetyl und der 2-Methoxypropionyl rest.

1-4C-Alkoxy-1-4C-alkoxy-1-4C-alkylcarbonyl steht für 1-4C-Alkoxy-1-4C-alkylcarbonyl, das im 1-4C-Alkoxyrest durch 1-4C-Alkoxy substituiert ist. Bevorzugt ist der Methoxy-ethoxy-methylcarbonyl rest.

1-4C-Alkoxycarbonylreste enthalten neben der Carbonyloxygruppe einen der vorstehend genannten 1-4C-Alkylreste. Bevorzugt sind der Methoxycarbonyl- und der Ethoxycarbonylrest.

1-4C-Alkoxy-1-4C-alkoxycarbonyl enthält neben der Carbonylgruppe einen an beliebiger Position durch 1-4C-Alkoxy substituierten 1-4C-Alkoxyrest. Bevorzugt ist der 2-Methoxy-ethoxy-carbonylrest.

Unter den Arylcarbonylresten sind der Benzoyl- und der 2-, 3- und 4-Methoxybenzoylrest bevorzugt.

Als bevorzugte Carbamoylreste seien beispielweise der Carbamoyl-, der Dimethylcarbamoyl-, der t-Butylcarbamoyl-, der 2-Hydroxyethylcarbamoyl-, der Phenyl carbamoyl-, der 4-Methoxyphenylcarbamoyl-,

der 2,4-Dimethoxyphenylcarbamoyl- und der Morpholinocarbonylrest genannt.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Tartrat, Amsonat, Embonat, Metembonat, Stearat, Tosylat, 2-Hydroxy-3-naphthoat, 3-Hydroxy-2-naphthoat oder Mesylat.

Gegenstand der Erfindung sind insbesondere solche Verbindungen der Formel I, worin das Brückensauerstoffatom -O- an ein Kohlenstoffatom der Alkylengruppe G1 geknüpft ist, das in direkter Nachbarschaft zu einem durch OR5 substituierten Kohlenstoffatom steht. Die erfindungsgemäßen Verbindungen haben in der Gruppierung G1 zumindest zwei asymmetrische Kohlenstoffatome. Die Erfindung umfaßt alle denkbaren Konfigurationskombinationen, beim Vorliegen zweier Asymmetriezentren also die R,R-, R,S-, S,R- und S,S-Kombination. Bevorzugter Gegenstand der Erfindung sind die optisch reinen Verbindungen der Formel I.

Hervorzuhebende erfindungsgemäße Verbindungen sind solche der Formel I, worin

R1 1-4C-Alkyl,

R2 1-4C-Alkyl, Formyl, Hydroxymethyl oder Cyanomethyl,

R3 Wasserstoff oder Halogen,

R4 Wasserstoff und

G1 eine substituierte Gruppe der Formel $-CH_2-CH(OR5)-CH_2-$bedeutet, in der

R5 1-4C-Alkyl, 1-4C-Alkoxy-1-4C-alkyl, Aryl-1-4C-alkyl, 1-4C-Alkylcarbonyl, 3-7C-Cycloalkylcarbonyl, Aryl-1-4C-alkylcarbonyl, 1-4C-Alkoxy-1-4C-alkylcarbonyl, 1-4C-Alkoxy-1-4C-alkoxy-1-4C-alkylcarbonyl, 1-4C-Alkoxycarbonyl, Arylcarbonyl, Furoyl oder eine Carbamoylgruppe der Formel

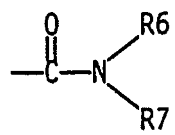

bedeutet, worin

R6 Wasserstoff (H), 1-4C-Alkyl, Hydroxy-1-4C-alkyl oder gemeinsam mit R7 und unter Einschluß des Stickstoffatoms, an das beide gebunden sind, Morpholino bedeutet und

R7 Wasserstoff (H), 1-4C-Alkyl, Aryl oder gemeinsam mit R6 und unter Einschluß des Stickstoffatoms, an das beide gebunden sind, Morpholino bedeutet, wobei

Aryl Phenyl oder substituiertes Phenyl mit einer, zwei oder drei 1-4C-Alkoxygruppen bedeutet,

und ihre Salze.

Die hervorzuhebenden erfindungsgemäßen Verbindungen können durch die folgende Formel I* charakterisiert werden,

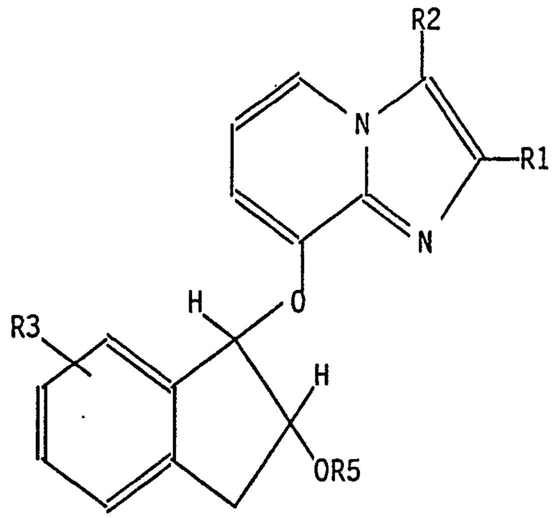

(I*)

worin R1, R2, R3 und R5 die für die hervorzuhebenden erfindungsgemäßen Verbindungen angegebenen Bedeutungen haben, und ihre Salze.

Ausgewählte erfindungsgemäße Verbindungen können durch die folgende Formel Ia charakterisiert werden,

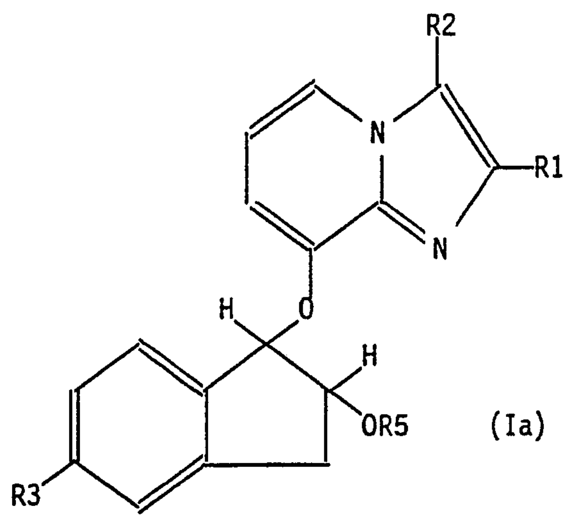

(Ia)

worin
R1 1-4C-Alkyl bedeutet,
R2 1-4C-Alkyl, Hydroxymethyl oder Cyanomethyl,
R3 Wasserstoff oder Halogen bedeutet und
R5 die für die hervorzuhebenden erfindungsgemäßen Verbindungen angegebenen Bedeutungen hat,
und ihre Salze.

Bevorzugt sind solche Verbindungen der Formel Ia, worin
R1 1-4C-Alkyl bedeutet,
R2 Hydroxymethyl oder Cyanomethyl,
R3 Wasserstoff oder Halogen bedeutet und
R5 die für die hervorzuhebenden erfindungsgemäßen Verbindungen angegebenen Bedeutungen hat,
und ihre Salze.

Unter Berücksichtigung der absoluten Konfiguration an den Positionen 1′ und 2′ im Dihydroindenylrest ergeben sich für die ausgewählten Verbindungen die folgenden besonders bevorzugten optisch reinen Konfigurationsisomeren,

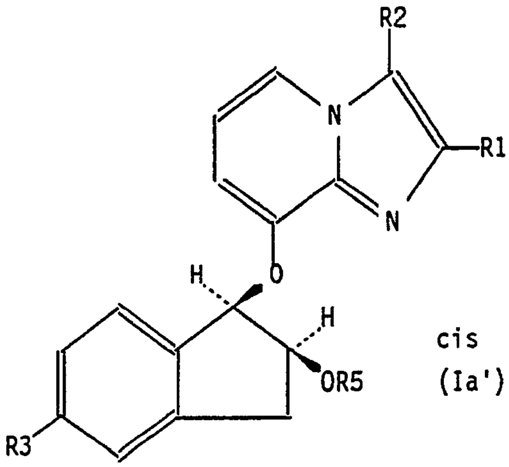

cis (Ia')

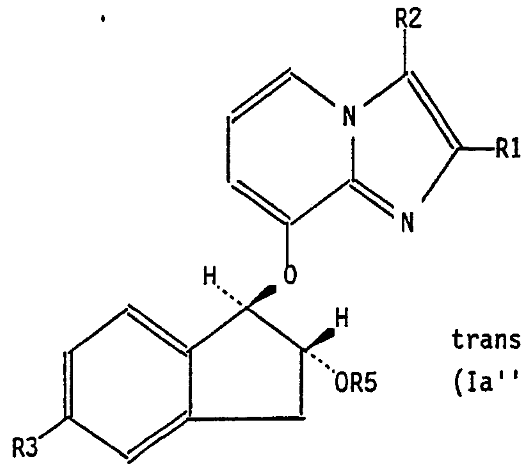

trans (Ia''')

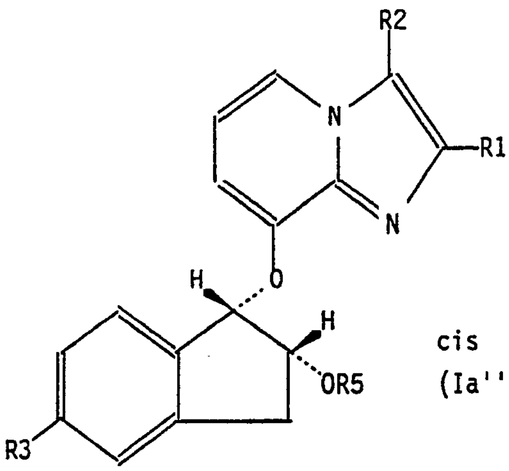

cis (Ia'')

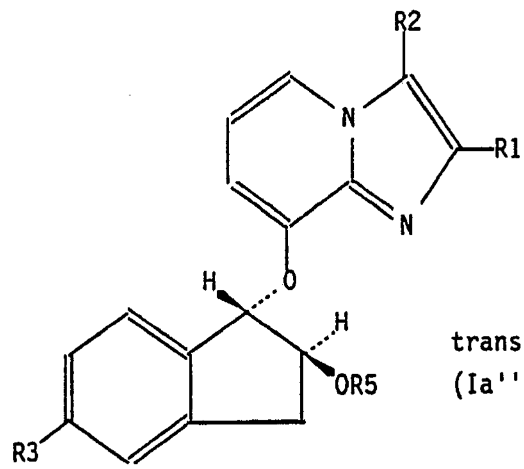

trans (Ia'''')

worin R1, R2, R3 und R5 die oben angegebenen Bedeutungen haben.

Beispielhafte ausgewählte erfindungsgemäße Verbindungen der Formel Ia sind mit ihren jeweiligen Substituentenbedeutungen in der folgenden Tabelle 1 wiedergegeben:

Tabelle 1

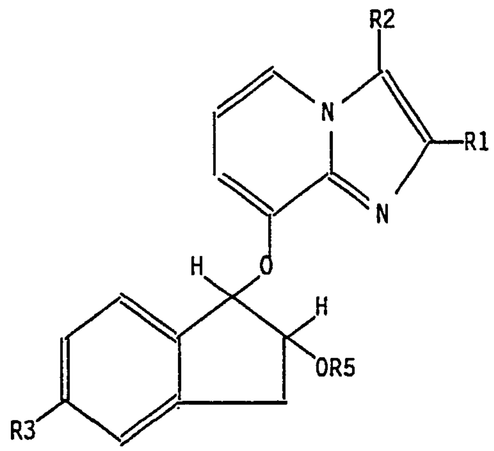

(Ia)

| R1 | R2 | R3 | R5 |
|---|---|---|---|
| $CH_3$ | $CH_2OH$ | F | $CO-CH_3$ |
| $CH_3$ | $CH_2OH$ | F | $CO-(CH_2)_3CH_3$ |
| $CH_3$ | $CH_2OH$ | H | CO– 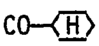 |
| $CH_3$ | $CH_2OH$ | Cl | $CO-CH_2OCH_3$ |
| $CH_3$ | $CH_2OH$ | F | $CO-CH_2OCH_2CH_2OCH_3$ |
| $CH_3$ | $CH_2OH$ | H | $CO-CH_2CH_2OCH_3$ |
| $CH_3$ | $CH_2OH$ | H | $CO-CH_2CH_2CH_2OCH_3$ |
| $CH_3$ | $CH_2OH$ | F | $CO-OCH_3$ |
| $CH_3$ | $CH_2OH$ | F | $CO-OCH_2CH_2OCH_3$ |
| $CH_3$ | $CH_2OH$ | H | $CO-OCH_2CH_2OCH_3$ |
| $CH_3$ | $CH_2OH$ | F | CO– 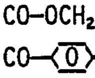 $-OCH_3$ |
| $CH_3$ | $CH_2OH$ | F | CO– 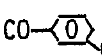 $OCH_3$ |
| $CH_3$ | $CH_2OH$ | F | CO– 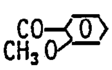 $CH_3O$ |
| $CH_3$ | $CH_2OH$ | F | CO– 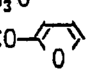 |
| $CH_3$ | $CH_3$ | H | $CO-CH_2OCH_3$ |

Fortsetzung Tabelle I

| R1 | R2 | R3 | R5 |
|---|---|---|---|
| $CH_3$ | $CH_3$ | F | $CO-CH_2OCH_3$ |
| $CH_3$ | $CH_2CN$ | H | $CO-CH_2OCH_3$ |
| $CH_3$ | $CH_2CN$ | F | $CO-CH_2OCH_3$ |
| $CH_3$ | $CH_2OH$ | H | $CH_2CH_3$ |
| $CH_3$ | $CH_2OH$ | H | $(CH_2)_3CH_3$ |
| $CH_3$ | $CH_2OH$ | F | $CH_3$ |
| $CH_3$ | $CH_2OH$ | Cl | $CH_3$ |
| $CH_2CH_3$ | $CH_2OH$ | H | $CH_3$ |
| $CH(CH_3)CH_3$ | $CH_2OH$ | F | $CH_3$ |
| $CH_3$ | $CH_2OH$ | H | $(CH_2)_3OCH_3$ |
| $CH_3$ | $CH_2OH$ | H | $(CH_2)_4OCH_3$ |
| $CH_3$ | $CH_2OH$ | H | $CH_2-C(CH_3)_2OCH_3$ |
| $CH_3$ | $CH_2OH$ | F | $CH_2CH_2OCH_3$ |
| $CH_2CH_3$ | $CH_2OH$ | H | $CH_2CH_2OCH_3$ |
| $CH_3$ | $CH_2OH$ | H | $CH_2CH_2OCH_3$ |
| $CH_3$ | $CH_2OH$ | H | 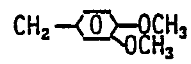 |
| $CH_3$ | $CH_2OH$ | F | 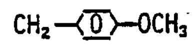 |
| $CH_3$ | $CH_2OH$ | F | 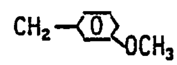 |
| $CH_3$ | $CH_2OH$ | F | 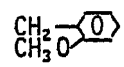 |
| $CH_3$ | $CH_2OH$ | F | 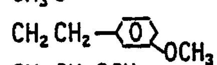 |
| $CH_3$ | $CH_3$ | F | $CH_2CH_2OCH_3$ |
| $CH_3$ | $CH_3$ | H | $CH_2CH_2OCH_3$ |
| $CH_3$ | $CH_3$ | H | $CH_3$ |
| $CH_3$ | $CH_2CN$ | H | $CH_2CH_2OCH_3$ |

Weiterer Gegenstand der Erfindung ist ein Verfahren zur Herstellung der erfindungsgemäßen Verbindungen und ihrer Salze. Das Verfahren ist dadurch gekennzeichnet, daß man in Verbindungen der Formel I, worin R1, R2, R3, R4 und G1 die oben angegebenen Bedeutungen haben und R5 Wasserstoff bedeutet (= Ausgangsverbindungen I), das Wasserstoffatom R5 durch einen Rest R5, der die eingangs angegebenen Bedeutungen hat, substituiert und gewünschtenfalls anschließend erhaltene Verbindungen der Formel I, worin R2 die Bedeutung Formyl hat, zu den Verbindungen der Formel I, worin R2 die Bedeutung Hydroxymethyl hat, reduziert, und/oder gewünschtenfalls anschließend erhaltene Salze in die freien Verbindungen oder erhaltene freie Verbindungen in die Salze überführt, wobei die Ausgangsverbindungen I als solche oder in Form ihrer Salze eingesetzt werden.

Die Substitution des Wasserstoffatoms R5 in den Ausgangsverbindungen I kann - je nach Art des

gewünschten Restes R5 - auf verschiedene Weise erfolgen.

So kann (a) der Rest R5 durch Umsetzung der Ausgangsverbindungen I mit Verbindungen der Formel IIa,

R5-Y (IIa)

worin R5 die eingangs angegebenen Bedeutungen hat und Y eine geeignete Fluchtgruppe (Abgangsgruppe) darstellt, eingeführt werden.

Wenn der gewünschte Rest R5 einen Carbamoylgruppe der Formel

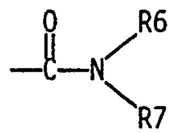

darstellt, in der R6 Wasserstoff (H) und R7 1-4C-Alkyl oder Aryl bedeutet, so kann dieser Rest auch (b) durch Umsetzung der Ausgangsverbindungen I mit Verbindungen der Formel IIb,

R7-N = C = O (IIb)

worin R7 1-4C-Alkyl oder Aryl bedeutet, hergestellt werden.

Wenn der gewünschte Rest R5 eine 1-4C-Alkoxycarbonylgruppe oder eine Carbamoylgruppe der Formel

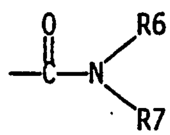

darstellt, so kann (c) seine Einführung auch in zwei Schritten erfolgen, indem man zunächst (beispielsweise mit Hilfe von Phosgen oder Trichlormethylchloroformiat) eine Chlorcarbonylgruppe in die Ausgangsverbindungen I einführt und anschließend mit einem 1-4C-Alkanol oder einem Amin der Formel IIc

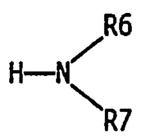

(IIc)

zum gewünschten Endprodukt umsetzt.

Welche Fluchtgruppe Y bei der Verfahrensvariante (a) geeignet ist, hängt von der Art des einzuführenden Substituenten R5 sowie von den weiteren Reaktionsbedingungen ab. Ist R5 eine substituierte Carbonylgruppe, so ist Y eine in Acylierungsreaktionen gebräuchliche Abgangsgruppe, beispielsweise ein Halogenatom (bevorzugt ein Chloratom), ein Rest -OR5 oder eine aus der Peptidchemie bekannte aktivierende Gruppe, wie sie intermediär bei der Umsetzung von Carbonsäuren mit Aktivierungsmitteln, wie beispielsweise Carbonyldiimidazol, Phosphoroxychlorid oder Chlorameisensäuremethylester, eingeführt wird . Ist R5 ein Alkyl-, Alkoxyalkyl- oder Arylalkylrest, so ist Y eine von Alkylierungsreaktionen bekannte Abgangsgruppe, beispielsweise ein Halogenatom, bevorzugt ein Brom-oder Jodatom oder beispielsweise eine Methylsulfonyl- oder Toluolsulfonylgruppe. Bei Alkylierungsreaktionen wird die Ausgangsverbindung I vorzugsweise nicht als solche, sondern in Form eines Salzes mit einer starken organischen oder anorganischen Base, insbesondere ihres Erdalkali- oder Alkalisalzes, mit der Verbindung IIa umgesetzt.

Die Umsetzung der Ausgangsverbindungen I mit den Verbindungen IIa, IIb oder IIc erfolgt beispielsweise in inerten, vorzugsweise polaren Lösungsmitteln, vorteilhafterweise in Gegenwart einer Base oder nach vorheriger Deprotonierung der Ausgangsverbindungen I. Die Umsetzung kann auch ohne Lösungsmittel oder unter Verwendung der eingesetzten Base als Lösungsmittel durchgeführt werden.

Die Umsetzung mit Acylierungsmitteln IIa und mit IIb werden vorteilhafterweise auch in Gegenwart von dem Fachmann bekannten Acylierungskatalysatoren, beispielsweise 4-Dimethylaminopyridin, durchgeführt. Die Umsetzung mit Alkylierungsmitteln IIa wird gegebenenfalls in Gegenwart bekannter Alkylierungskatalysatoren, beispielsweise Phasentransferkatalysatoren, Kronenethern und/oder Alkaliiodiden, durchgeführt.

Die Reaktionstemperatur richtet sich nach der Reaktivität der eingesetzten Verbindungen II bzw. nach der Effektivität der verwendeten Hilfsbase. Im allgemeinen kann die Reaktionstemperatur zwischen 0° und

der Siedetemperatur des verwendeten Lösungsmittels betragen.

Die Reduktion der Verbindungen I, worin R2 die Bedeutung Formyl hat, zu den Verbindungen I, worin R2 die Bedeutung Hydroxymethyl hat, erfolgt auf eine dem Fachmann geläufige Weise, z.B. durch Umsetzung der Formylverbindung mit Natriumborhydrid. Hierbei kommen als Lösungsmittel Wasser, niedere Alkohole, wie Methanol und Ethanol, Ether, wie Tetrahydrofuran und Dioxan, und inerte Lösungsmittel, wie Dimethylformamid, in Frage. Zum Schutz von Acylgruppen kann der pH auch im Bereich von 6 - 9 konstant gehalten werden. Im Falle von Verbindungen I mit Etherresten -OR5 können auch stärkere Reduktionsmittel, wie beispielsweise Lithiumaluminiumhydrid oder Natrium-bis-(2-methoxyethoxy)-dihydroaluminat in inerten Lösungsmitteln, wie Tetrahydrofuran oder Toluol zur Anwendung kommen.

Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylen chlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Enantiomer reine Endprodukte I erhält man durch Umsetzung enantiomer reiner Ausgangsverbindungen I mit den Verbindungen IIa, IIb oder IIc.

Die Ausgangsverbindungen der Formel I, in denen R5 Wasserstoff bedeutet, erhält man, indem man Imidazopyridine der Formel III mit Bicyclen der Formel IV umsetzt,

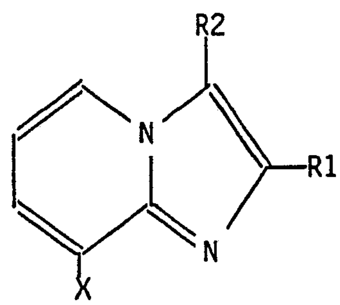 (III) 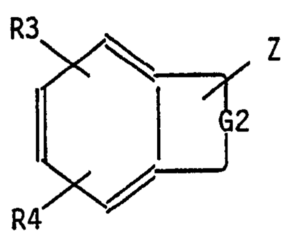 (IV)

und gewünschtenfalls anschließend erhaltene Verbindungen der Formel I mit R5 = H, worin R2 die Bedeutung Formyl hat, zu den Verbindungen der Formel I, worin R2 die Bedeutung Hydroxymethyl hat, reduziert, wobei R1, R2, R3 und R4 die oben angegebenen Bedeutungen haben, G2 gemeinsam mit Z eine Gruppierung darstellt, die mit X unter Bildung der Gruppen -O- (Sauerstoff) und G1 zu reagieren in der Lage ist, oder G2 die Bedeutung von G1 hat und X und Z geeignete reaktive Gruppen darstellen, die unter Bildung der Gruppe -O- (Sauerstoff) zu reagieren in der Lage sind, wobei G1 eine substituierte Gruppe der Formel $-CH(OH)-CH_2-CH_2-$, $-CH_2-CH(OH)-CH(CH_3)-$, $-CH_2-CH(OH)-CH_2-$, $-CH(OH)-CH_2-CH_2-CH_2-$, $-CH_2-CH(OH)-CH_2-CH_2$, $-CH(OH)-CH_2-CH_2-CH_2-CH_2-$ oder $-CH_2-CH(OH)-CH_2-CH_2-CH_2-$ bedeutet.

Für die Herstellung erfindungsgemäßer Verbindungen der Formel I, in denen R2 Hydroxymethyl bedeutet, werden bevorzugt Ausgangsverbindungen der Formel I eingesetzt, in denen R2 die Bedeutung Formyl hat.

Die Art der Umsetzung der Imidazopyridine III mit den Bicyclen IV hängt von den Strukturen der (reaktiven) Gruppen G2, X und Z ab. Folgende (reaktive) Gruppen seien beispielsweise genannt:

X = OH Z = Halogen G2 = G1

oder X = OH G2 und Z gemeinsam stellen eine 1,2-Epoxitrimethylen-, 1,2- oder 2,3-Epoxitetramethylen- oder 1,2- oder 2,3-Epoxipentamethylengruppe dar.

Die Reaktion von III mit IV wird in geeigneten, inerten Lösungsmitteln durchgeführt, wobei die

Kondensation unter Abspaltung von H-Halogen die Gegenwart einer geeigneten Base bzw. die vorherige Deprotonierung (z.B. mit einem Hydrid, wie Natriumhydrid) erfordert. Werden von den Bicyclen IV nicht die Halogenalkohole sondern die Epoxide eingesetzt, so erfolgt die Umsetzung mit den Imidazopyridinen III in Gegenwart von Aluminiumoxid oder bevorzugt in polaren, protonenhaltigen Medien in Gegenwart von Basen. Als protonenhaltige Medien kommen Wasser und Alkohole, vorzugsweise Methanol, alleine oder in Zumischung in Frage. Als Basen seien Alkali- oder Erdalkalihydroxide, vorzugsweise Alkalicarbonate und tertiäre organische Amine genannt. Sie können je nach Art im Überschuß oder Unterschuß eingesetzt werden, vorzugsweise in einer Menge von 0,1 - 2 Mol. Die Umsetzung in polaren Medien wird bei Temperaturen von 0° bis 100 °C, vorzugsweise bei 20° bis 60°C durchgeführt.

Die Herstellung der Epoxide aus geeigneten Vorstufen) z.B. Halogenalkoholen, kann auch in situ erfolgen und im Eintopfverfahren mit deren Umsetzung mit Verbindungen III kombiniert werden, indem wenigstens ein weiteres Mol Base zur Anwendung kommt. - Die Herstellung der Ausgangsverbindungen I mit R5 = H erfolgt bevorzugt durch Umsetzung der Imidazopyridine III (mit X = OH) mit 1,2-Epoxiden, wobei fast ausschließlich das trans-Additionsprodukt entsteht.

Welche Reaktionsbedingungen für die Herstellung der Ausgangsverbindungen I im einzelnen erforderlich sind, ist dem Fachmann aufgrund seines Fachwissens geläufig.

Enantiomer reine Ausgangsverbindungen I erhält man beispielsweise durch Umsetzung enantiomer reiner Bicyclen IV mit den Imidazopyridinen III. Die enantiomerenreinen Ausgangsverbindungen I mit der trans-Konfiguration (entsprechend den Endprodukten Ia‴ und Ia⁗) erhält man als trans-Additionsprodukte bevorzugt durch Umsetzung der entsprechenden enantiomerenreinen Epoxide mit den Imidazopyridinen III. Die (enantiomerenreinen) Ausgangsverbindungen I mit cis-Konfiguration (entsprechend den Endprodukten Ia′ und Ia″) sind beispielsweise durch Epimerisierung der trans-Konfigurationsisomeren zugänglich. Die Epimerisierung wird beispielweise - ausgehend vom entsprechenden Mesylat - durch Reaktion mit Kaliumacetat in Gegenwart eines Kronenethers und anschließende Verseifung durchgeführt.

Die Ausgangsverbindungen III sind literaturbekannt oder können in Analogie zu literaturbekannten Methoden, beispielsweise in Analogie zu J.J.Kaminski et al. [J.Med.Chem. 28, 876 (1985)] oder wie in den Europäischen Patentanmeldungen 33 094 oder 68 378 beschrieben hergestellt werden. Die Ausgangsverbindungen IV sind ebenfalls literaturbekannt [siehe z.B. G.H. Posner et al., J. Amer. Chem. Soc. 99, 8214 (1977); M.N. Akhtar et al., J. Chem. Soc. Perk. Trans. I (1979) 2437; D.R. Boyd et al., J. Chem. Soc. Perk. Trans. I (1982) 2767 sowie Bull. Soc. Chim. (France) 11, 3092-3095 (1973)] oder sie können ebenfalls in Analogie zu literaturbekannten Methoden hergestellt werden.

Bevorzugte Ausgestaltungen des Verfahrens sind solche, bei denen die Substituenten und Symbole R1, R2, R3, R4 und G1 die in den Unter- und Nebenansprüchen angegebenen Bedeutungen haben.

Die folgenden Beispiele erläutern die Erfindung näher ohne sie einzuschränken. Die in den Beispielen namentlich aufgeführten Verbindungen der allgemeinen Formel I sowie die Salze dieser Verbindungen sind bevorzugter Gegenstand der Erfindung. Schmp. bedeutet Schmelzpunkt, für Stunde(n) wird die Abkürzung h und für Minuten die Abkürzung Min. verwendet. Unter "Ether" wird Diethylether verstanden.

## Beispiele

### 1. 8-(trans-2-Acetoxy-2,3-dihydro-1-indenyloxy)-3-hydroxymethyl-2-methyl-imidazo[1,2-a]pyridin

Man löst 0,20 g 8-(trans-2-Acetoxy-2,3-dihydro-1-indenyloxy)3-formyl-2-methyl-imidazo[1,2-a]pyridin in der Wärme in 15 ml Methanol, setzt bei Raumtemperatur überschüssiges 97 %iges Natriumborhydrid (11,1 mg) zu und neutralisiert nach ca. 20 Min. mit Eisessig. Man destilliert am Rotavapor das Lösungsmittel ab, versetzt mit Wasser, extrahiert mit Dichlormethan und destilliert im Vakuum das Lösungsmittel ab. Der Rückstand (0,22 g) wird aus Ethylacetat/Diisopropylether (- 20°C) umkristallisiert. Man erhält die Titelverbindung vom Schmp. 198 - 199°C (Zersetzung).

### 2. 3-Hydroxymethyl-8-[trans-2-(4-methoxybenzoyloxy)-2,3-dihydro-1-indenyloxy]-2-methyl-imidazo[1,2-a]-pyridin

Man versetzt 1,0 g 3-formyl-8-[trans-2-(4-methoxybenzoyloxy)-2,3-dihydro-1-indenyloxy]-2-methyl-imidazo[1,2-a]pyridin, gelöst in 170 ml Methanol, bei 25°C portionsweise mit soviel überschüssigem

Natriumborhydrid (ca. 180 mg), bis die Reaktion beendet ist. Man arbeitet analog Beispiel 1 auf und erhält in 85 % Ausbeute die Titelverbindung vom Schmp. 199,5 - 200°C unter Zersetzung (aus Chlorbenzol).

3. 8-[trans-2-(2,6-Dimethoxybenzoyloxy)-2,3-dihydro-1-indenyloxy]-3-hydroxymethyl-2-methyl-imidazo[1,2-a]pyridin

Man versetzt 1,0 g 3-Formyl-8-[trans-2-(2,6-dimethoxybenzoyloxy)-2,3-dihydro-1-indenyloxy]-2-methyl-imidazo[1,2-a]pyridin, gelöst in einer Mischung von 40 ml Dimethylformamid und 10 ml Methanol, bei 60°C portionsweise mit überschüssigem Natriumborhydrid, fällt mit Wasser und kristallisiert den Niederschlag aus Dioxan um. Man erhält in 82 % Ausbeute die Titelverbindung vom Schmp. 230 - 231°C (Zersetzung).

4. 3-Hydroxymethyl-2-methyl-8-[trans-2-(3,4,5-trimethoxybenzoyloxy)-2,3-dihydro-1-indenyloxy]-imidazo[1,2-a]pyridin

Zu einer Lösung von 0,5 g 3-formyl-2-methyl-8-[trans-2-(3,4,5-trimethoxybenzoyloxy)-2,3-dihydro-1-indenyloxy]-imidazo[1,2-a]pyridin in 20 ml Tetrahydrofuran und 5 ml Wasser tropft man eine Lösung von 78 mg 97 %igem Natriumborhydrid in 2 ml Wasser und hält pH 9 durch gleichzeitiges Zutropfen von 0,3 %iger Essigsäure. Man arbeitet analog Beispiel 1 auf und erhält in 97 % Ausbeute die Titelverbindung vom Schmp. 199 - 200°C unter Zersetzung (aus Ethanol/Toluol).

5. 8-(5-Fluor-trans-2-methoxyacetoxy-2,3-dihydro-1-indenyloxy)-3-hydroxymethyl-2-methyl-imidazo[1,2-a]-pyridin

Zu einer Lösung von 12,0 g 8-(5-Fluor-trans-2-methoxyacetoxy-2,3-dihydrol-1-indenyloxy)-3-formyl-2-methyl-imidazo[1,2-a]pyridin in 600 ml Methanol und 30 ml Wasser werden bis zur vollständigen Umsetzung 5,3 g 97 %iges Natriumborhydrid, gelöst in 50 ml Wasser, und gleichzeitig eine 10 %ige Lösung von Eisessig in Methanol so zugetropft, daß pH 8 gehalten wird. Man arbeitet analog Beispiel 1 auf und erhält 10,0 g (83 %) der Titelverbindung vom Schmp. 185 - 186°C unter Zersetzung (aus Ether).

Die nachfolgenden Verbindungen 6 - 14 werden in ähnlicher Weise wie im Beispiel 1 beschrieben durch Reduktion entsprechender 3-Formyl-imidazo[1,2-a]pyridine mit Natriumborhydrid hergestellt:

6. 8-(trans-2-Cyclopropylcarbonyloxy-2,3-dihydro-1-indenyloxy)-3-hydroxymethyl-2-methylimidazo[1,2-a]-pyridin

Schmp. 185 - 187°C, aus Ethylacetat.

7. 3-Hydroxymethyl-2-methyl-8-(trans-2-phenylacetoxy-2,3-dihydro-1-indenyloxy)-imidazo[1,2-a]pyridin

Schmp. 163 - 165°C, aus Ether.

8. 8-(trans-2-Furoyloxy-2,3-dihydro-1-indenyloxy)-3-hydroxymethyl-2-methyl-imidazo[1,2-a]pyridin

Schmp. 199 - 200°C (Zersetzung), aus Ether.

9. 8-(trans-2-Dimethylcarbamoyloxy-2,3-dihydro-1-indenyloxy)-3-hydroxymethyl-2-methyl-imidazo[1,2-a]-pyridin

Schmp. 177 - 179°C, aus Ether.

10. 8-(trans-2-Carbamoyloxy-2,3-dihydro-1-indenyloxy)-3-hydroxymethyl-2-methyl-imidazo[1,2-a]pyridin

Schmp. 196 - 198°C (Zersetzung), aus Acetonitril.

11. 8-[trans-2-(2-Hydroxyethylcarbamoyloxy)-2,3-dihydro-1-indenyloxy]-3-hydroxymethyl-2-methyl-imidazo-

[1,2-a]pyridin x 1/2 Ethanol

Schmp. 196 - 197°C (Zersetzung), aus Ethanol.

12. 8-(trans-2-tert-Butylcarbamoyloxy-2,3-dihydrol-indenyloxy)-3-hydroxymethyl-2-methyl-imidazo[1,2-a]-pyridin

Schmp. 205 - 206°C (Zersetzung), aus Cyclohexan/Toluol.

13. 8-[trans-2-(N-2-Hydroxyethyl-N-methyl-carbamoyloxy)-2,3-dihydro-1-indenyloxy]-3-hydroxymethyl-2-methyl-imidazo[1,2-a]pyridin

Die Verbindung wird durch Chromatographie an Kieselgel mit Dichlormethan/Methanol (95:5) gereinigt. Schmp. 163 - 164°C (Zersetzung), aus Ether.

14. 3-Hydroxymethyl-8-(trans-2-methoxy-2,3-dihydro-1-indenyloxy)-2-methyl-imidazo[1,2-a]pyridin

Schmp. 98 - 100°C, aus Diisopropylether.

Die nachfolgenden Verbindung 15 - 22 werden in ähnlicher Weise wie in Beispiel 2 beschrieben durch Reduktion entsprechender 3-Formyl-imidazo[1,2-a]pyridine mit Natriumborhydrid hergestellt:

15. 8-(trans-2-Benzoyloxy-2,3-dihydro-1-indenyloxy)-3-hydroxymethyl-2-methyl-imidazo[1,2-a]pyridin

Schmp. 193 - 195°C (Zersetzung), aus Ethylacetat.

16. 3-Hydroxymethyl-8-[trans-2-(3-methoxybenzoyloxy)-2,3-dihydro-1-indenyloxy]-2-methyl-imidazo[1,2-a]-pyridin

Schmp. 163 - 165°C, aus n-Butanol/Petrolether.

17. 3-Hydroxymethyl-8-[trans-2-(2-methoxybenzoyloxy)-2,3-dihydro-1-indenyloxy]-2-methyl-imidazo[1,2-a]-pyridin

Schmp. 181 - 182°C (Zersetzung), aus n-Butanol/Petrolether.

18. 8-[trans-2-(3,4-Dimethoxybenzoyloxy)-2,3-dihydro-1-indenyloxy]-3-hydroxymethyl-2-methylimidazo[1,2-a]pyridin

Schmp. 190 - 192°C (Zersetzung), aus Ether.

19. 3-Hydroxymethyl-2-methyl-8-(trans-2-phenylcarbamoyloxy-2,3-dihydro-1-indenyloxy)-imidazo[1,2-a]-pyridin

Schmp. 181 - 183°C (Zersetzung), aus Ethanol/Ether.

20. 3-Hydroxymethyl-2-methyl-8-(trans-2-morpholinocarbonyloxy-2,3-dihydro-1-indenyloxy)-imidazo[1,2-a]-pyridin

Schmp. 146 - 148°C, aus Toluol.

21. 3-Hydroxymethyl-8-[trans-2-(4-methoxyphenylcarbamoyloxy)-2,3-dihydro-1-indenyloxy]-2-methyl-imidazo[1,2-a]pyridin

Schmp. 166 - 167°C (Zersetzung), aus Toluol.

22. 8-[trans-2-(2,4-Dimethoxyphenylcarbamoyloxy)-2,3-dihydro-1-indenyloxy]-3-hydroxymethyl-2-methyl-imidazo[1,2-a]pyridin

Schmp. 170 - 172°C (Zersetzung), aus 2-Methoxyethanol.

Die nachfolgenden Verbindung 23 - 28 werden in ähnlicher Weise wie in Beispiel 5 beschrieben durch Reduktion entsprechender 3-Formyl-imidazo[1,2-a]pyridine mit Natriumborhydrid bei pH 8 hergestellt:

23. 3-Hydroxymethyl-8-[trans-2-(methoxyacetoxy)-2,3-dihydro-1-indenyloxy]-2-methyl-imidazo[1,2-a]pyridin

Schmp. 168 - 169°C (Zersetzung), aus Toluol.

24. (-)-3-Hydroxymethyl-8-{trans-2-[(2S)-methoxypropionyloxy]-2,3-dihydro-1-indenyloxy}-2-methyl-imidazo-[1,2-a]pyridin

Die Reaktion ist mit 2,2 mol wäßriger Natriumborhydrid-Lösung in Methanol bei 5 - 10°C und pH 8 in ca. 10 Min. beendet. Schmp. 144 - 145°C, aus Ether;
$\alpha_D^{20}$ : 28,2° (c = 1, Ethanol).

25. (+)-3-Hydroxymethyl-8-{trans-2-[(2R)-methoxypropionyloxy]-2,3-dihydro-1-indenyloxy}-2-methyl-imidazo[1,2-a] pyridin

Durchführung analog Beispiel 24. Schmp. 144 - 145°C, aus Ether;
$\alpha_D^{20}$ : + 28,0° (c = 1, Ethanol).

26. (-)-8-{5-Fluor-trans-2-[(2S)-methoxypropionyloxy]-2,3-dihydro-1-indenyloxy}-3-hydroxymethyl-2-methyl-imidazo[1,2-a]pyridin

Durchführung analog Beispiel 24. Schmp. 162 - 163°C, aus Ether;
$\alpha_D^{20}$ : - 27,1° (c = 1, Ethanol).

27. 3-Hydroxymethyl-8-(trans-2-methoxycarbonyloxy-2,3-dihydro-1-indenyloxy)-2-methyl-imidazo[1,2-a]-pyridin

Schmp. 175 - 176°C (Zersetzung), Ether.

28. 3-Hydroxymethyl-8-[trans-2-(2-methoxyethoxyacetoxy)-2,3-dihydro-1-indenyloxy]-2-methyl-imidazo[1,2-a]pyridin

Schmp. 152 - 154°C, aus Ether.

29. 8-(trans-2-Acetoxy-2,3-dihydro-1-indenyloxy)-3-formyl-2-methyl-imidazo[1,2-a]pyridin

Zu 3,0 g 3-Formyl-8-(trans-2-hydroxy-2,3-dihydro-1-indenyloxy)-2-methyl-imidazo[1,2-a]pyridin und 0,1 Moläquivalent (119 mg) 4-Dimethylaminopyridin in 30 ml Pyridin tropft man bei Raumtemperatur 5 Moläquivalent (5,0 g) Essigsäureanhydrid. Nach 3 h setzt man 4 Moläquivalent Wasser zu, rührt 30 Min., gießt die Reaktionslösung auf Eiswasser und extrahiert mit Ethylacetat. Man wäscht die organische Phase mit Kaliumcarbonatlösung und Wasser, engt sie im Vakuum ein und kristallisiert den Rückstand aus Ethanol um. Man erhält die Titelverbindung in 85 % Ausbeute. Schmp. 145,5 - 147°C.

30. 8-(trans-2-Benzoyloxy-2,3-dihydro-1-indenyloxy)-3-formyl-2-methyl-imidazo[1,2-a]pyridin

Die Herstellung erfolgt analog Beispiel 29 unter Verwendung von Benzoesäureanhydrid. Schmp. 174 - 175°C (aus Methanol).

31. 3-Formyl-8-[trans-2-(4-methoxybenzoyloxy)-2,3-dihydro-1-indenyloxy]-2-methyl-imidazo[1,2-a]pyridin

Zu 10,0 g 3-Formyl-8-(trans-2-hydroxy-2,3-dihydro-1-indenyloxy)-2-methy-imidazo[1,2-a]pyridin und 4,0 g 4-Dimethylaminopyridin in 100 ml Pyridin tropft man unter Kühlung mit Eis 11,2 g 4-Methoxybenzoylchlorid und rührt noch 24 h bei Raumtemperatur. Man gießt auf 500 ml Eiswasser und trennt das Reaktionsprodukt durch Filtrieren oder Ausschütteln mit Ethylacetat und Waschen der organischen Phase mit verdünnter Salzsäure, Natriumcarbonatlösung und Wasser und Konzentrieren im Vakuum ab. Man erhält in 80 - 86 % die Titelverbindung. Schmp. 182 - 183°C (aus Toluol).

In ähnlicher Weise wie in Beispiel 31 beschrieben erhält man aus dem gleichen Imidazo[1,2-a]pyridin durch Umsetzung mit Cyclopropancarbonsäurechlorid, Phenylacetylchlorid, 2-Furancarbonsäurechlorid, 3,4,5-Trimethoxybenzoylchlorid, 3-Methoxybenzoylchlorid, 2-Methoxybenzoylchlorid und 3,4-Dimethoxybenzoylchlorid die nachfolgenden Verbindungen 32 - 38:

32. 8-(trans-2-Cyclopropylcarbonyloxy-2,3-dihydro-1-indenyloxy)-3-formyl-2-methyl-imidazo[1,2-a]pyridin

Schmp. 119 - 121°C, aus Ether und Petrolether (100 - 140°C).

33. 3-Formyl-2-methyl-8-(trans-2-phenylacetoxy-2,3-dihydro-1-indenyloxy)-imidazo[1,2-a]pyridin

Man erhält nach Chromatographie an Kieselgel mittels Dichlormethan/Ethylacetat (9:1) und Verreiben mit Ether ein Ether enthaltendes Produkt vom Schmp. 111 -113°C, das ohne weitere Reinigung in Beispiel 7 eingesetzt wird.

34. 3-Formyl-8-[trans-2-(2-furoyloxy)-2,3-dihydrol-indenyloxy]-2-methyl-imidazo[1,2-a]pyridin

Schmp. 182 - 184°C (aus Toluol).

35. 3-Formyl-2-methyl-8-[trans-2-(3,4,5-trimethoxybenzoyloxy)-2,3-dihydro-1-indenyloxy]-imidazo[1,2-a]-pyridin

Schmp. 179 - 180°C (aus Toluol).

36. 3-Formyl-8-[trans-2-(3-methoxybenzoyloxy)-2,3-dihydro-1-indenyloxy]-2-methyl-imidazo[1,2-a]pyridin

Schmp. 150 - 151°C (aus Toluol).

37. 3-Formyl-8-[trans-2-(2-methoxybenzoyloxy)-2,3-dihydro-1-indenyloxy]-2-methyl-imidazo[1,2-a]pyridin

Schmp. 144 - 146°C (aus Toluol).

38. 8-[trans-2-(3,4-Dimethoxybenzoyloxy)-2,3-dihydrolindenyloxy]-3-formyl-2-methyl-imidazo[1,2-a]pyridin

Nach Chromatographie an Kieselgel mit Ethylacetat/Petrolether (50 - 70°C) 4:1.
Schmp. 195 - 196°C (Zersetzung).

39. 8-[trans-2-(2,6-Dimethoxybenzoyloxy)-2,3-dihydro-1-indenyloxy]-3-formyl-2-methyl-imidazo[1,2-a]pyridin

1,5 g 3-Formyl-8-(trans-2-hydroxy-2,3-dihydro-1-indenyloxy)-2-methyl-imidazo[1,2-a]pyridin, 7,5 g 4-Dimethylaminopyridin und 2,0 g 2,6-Dimethoxybenzoylchlorid werden 1 h auf 120 - 130°C erhitzt. Man verdünnt mit 100 ml Dichlormethan, extrahiert mit verdünnter Salzsäure, engt die organische Phase im Vakuum ein und chromatographiert den Rückstand mit Ethylacetat/Isopropylalkohol (9:1) an Kieselgel. Man erhält 2,0 g (89 %) der Titelverbindung, die aus Toluol und Methanol umkristallisiert wird. Schmp. 180 - 181°C.

40. 8-(5-Fluor-trans-2-methoxyacetoxy-2,3-dihydro-1-indenyloxy)-3-formyl-2-methyl-imidazo[1,2-a]pyridin

Man löst 13,0 g 8-(5-Fluor-trans-2-hydroxy-2,3-dihydro-1-indenyloxy)-3-formyl-2-methyl-imidazo[1,2-a]-

pyridin und 5 g 4-Dimethylaminopyridin in 140 ml Pyridin bei 50 - 60°C und tropft bei 10°C 8,8 g Methoxyacetylchlorid zu. Man rührt noch 3 h bei Raumtemperatur, gießt auf Eis und stellt mit konzentrierter Salzsäure auf pH 6. Den ausgefallenen Niederschlag (14 g) kristallisiert man aus Toluol und Ether um und erhält die Titelverbindung vom Schmp. 114 - 116°C.

41. 3-Formyl-8-(trans-2-methoxyacetoxy-2,3-dihydro-1-indenyloxy)-2-methyl-imidazo[1,2-a]pyridin

Man erhält die Titelverbindung vom Schmp. 106 - 108°C [aus Ethylacetat/Petrolether (100 - 140°C)] analog Beispiel 40 aus 3-Formyl-8-(trans-2-hydroxy-2,3-dihydro-1-indenyloxy)-2-methyl-imidazo[1,2-a]-pyridin.

42. 3-Cyanomethyl-8-(trans-2-methoxyacetoxy-2,3-dihydro-1-indenyloxy)-2-methyl-imidazo[1,2-a]pyridin

Man erhält die Titelverbindung vom Schmp. 132 - 133°C [aus Petrolether (40 -60° C)] analog Beispiel 40 aus 3-Cyanomethyl-8-(trans-2-hydroxy-2,3-dihydro-1-indenyloxy)-2-methyl-imidazo[1,2-a]pyridin und Methoxyacetylchlorid und Chromatographie an Kieselgel mit Ethylacetat/Methanol 4:1.

43. (-)-3-Formyl-8-{trans-2-[(2S)-methoxypropionyloxy]-2,3-dihydro-1-indenyloxy}-2-methyl imidazo[1,2-a]-pyridin

a) Analog Freudenberg und Markert, Chem. Ber. 60B, 2447 (1927) tropft man zu 16,5 mol 45 %iger Kaliumhydroxidlösung bei 4 - 8°C 80,5 ml (1,08 mol) (S)-(+)-Milchsäure (d = 1.206) und während 8 h 1393 ml Dimethylsulfat, wobei zum Schluß heftige Gasentwicklung (Dimethylether) einsetzt. Man versetzt mit 2 l Eiswasser und konzentrierter Salzsäure bis pH 1,4 und extrahiert die Lösung im Perforator mit Dichlormethan. Durch fraktionierte Destillation erhält man 32 g (2S)-Methoxypropionsäure, Sdp. 92 - 93°C (7 mbar) und $n_D^{20}$ : - 83,8° (neat).

b) Zu 1,5 g der Säure in 20 ml Dichlormethan setzt man bei 10°C 1 Tropfen Dimethylformamid und 2 ml Oxalylchlorid zu und rührt bei Raumtemperatur bis zur Beendigung der Gasentwicklung. Man engt im Vakuum ein, wiederholt dies nach Zugabe von wasserfreiem Dichlormethan und tropft den mit Dichlormethan verdünnten Rückstand von (2S)-Methoxypropionylchlorid bei 10°C zu 2,2 g 3-Formyl-8-(trans-2-hydroxy-2,3-dihydro-1-indenyloxy]-2-methyl-imidazo[1,2-a]pyridin und 1,8 g 4-Dimethylaminopyridin in 20 ml Pyridin. Man rührt 5 h bei Raumtemperatur und reinigt nach üblicher Aufarbeitung durch Chromatographie an Kieselgel mit Ethylacetat/Petrolether (4:1). Man erhält nach Umkristallisieren aus Methanol 1,35 g (48 %) der Titelverbindung vom Schmp. 65 - 66°C.

44. (-)-8-{5-Fluor-trans-2-[(2S)-methoxypropionyloxy]-2,3-dihydro-1-indenyloxy}-3-formyl-2-methyl-imidazo-[1,2-a]pyridin

Die Titelverbindung vom Schmp. 95 - 97°C (Chromatographie an Kieselgel mit Ethylacetat/Petrolether 2:1) wird analog Beispiel 43 aus 8-(5-Fluor-trans-2-hydroxy-2,3-dihydro-1-indenyloxy)-3-formyl-2-methyl-imidazo[1,2-a]pyridin und (2S)-Methoxypropionylchlorid hergestellt.

45. (+)-3-Formyl-8-{trans-2-[(2R)-methoxypropionyloxy]-2,3-dihydro-1-indenyloxy}-2-methyl-imidazo[1,2-a]-pyridin

130 g (R)-(+)-Milchsäureisobutylester (97 %) werden bei 10°C in 14,2 mol 45 %ige Kaliumhydroxidlösung getropft. Man rührt 2 h bei dieser Temperatur und extrahiert die Lösung zweimal mit jeweils 100 ml Diisopropylether. Die wäßrige Lösung von (R)-Natriumlactat wird analog Beispiel 43 mit 1180 ml Dimethylsulfat umgesetzt. Man erhält 31 g (2R)-Methoxypropionsäure, Sdp. 94 - 95°C (8 mbar) und $n_D^{20}$ : + 85,0° - (neat). Die weitere Umsetzung zur Titelverbindung, die in 60 % Ausbeute als nicht kristallisierendes Öl anfällt, erfolgt analog Beispiel 43.

46. 3-Formyl-8-[trans-2-(2-methoxyethoxyacetoxy)-2,3-dihydro-1-indenyloxy]-2-methyl-imidazo[1,2-a]pyridin

1,5 g 2-Methoxyethoxyessigsäure (Chem. Ber. 63B, 3117) werden mit 2,5 g Oxalylchlorid analog Beispiel 43 b) zum rohen Säurechlorid und dieses weiter mit 3-Formyl-8-(trans-2-hydroxy-2,3-dihydro-1-indenyloxy)-2-methyl-imidazo[1,2-a]pyridin zur Titelverbindung umgesetzt. Schmp. 88 - 90°C nach Chromatographie an Kieselgel mit Dichlormethan/Methanol 20:1.

47. 3-Formyl-8-(trans-2-methoxycarbonyloxy-2,3-dihydro-1-indenyloxy)-2-methyl-imidazo[1,2-a]pyridin

1,5 g 3-Formyl-8-(trans-2-hydroxy-2,3-dihydro-1-indenyloxy)-2-methyl-imidazo[1,2-a]pyridin, 1,9 g Chlorameisensäuremethylester, 0,6 g 4-Dimethylaminopyridin und 10 ml Pyridin werden 8 h bei 60°C gerührt. Man versetzt mit Wasser und Dichlormethan, säuert mit konzentrierter Salzsäure auf pH 5 an und arbeitet die organische Phase durch Chromatographie an Kieselgel mit Ethylacetat/Cyclohexan 4:1 auf. Man erhält in 50 % Ausbeute die Titelverbindung vom Schmp. 137 -139°C.

48. 3-Formyl-8-(trans-2-methoxy-2,3-dihydro-1-indenyloxy)-2-methyl-imidazo-[1,2-a]pyridin

Zu einer Lösung von 2,0 g 3-Formyl-8-(trans-2-hydroxy-2,3-dihydro-1-indenyloxy)-2-methyl-imidazo-[1,2a]pyridin in 20 ml Tetrahydrofuran fügt man bei Raumtemperatur 1,3 g gepulvertes Kaliumhydroxyd und 1,2 ml Methyliodid zu und rührt 24 h bei Raumtemperatur. Man gießt die Mischung auf Eis, extrahiert bei pH 7 mit Dichlormethan und chromatographiert an Kieselgel mit Toluol/Dioxan (4:1). Man erhält 1,5 g der Titelverbindung vom Schmp. 179 - 180°C.

49. 3-Formyl-8-(trans-2-dimethylcarbamoyloxy-2,3-dihydro-1-indenyloxy)-2-methyl-imidazo[1,2-a]pyridin

Man erhitzt 1,5 g 3-Formyl-8-(trans-2-hydroxy-2,3-dihydro-1-indenyloxy]-2-methyl-imidazo[1,2-a]pyridin, 1,2 g Dimethylcarbamoylchlorid und 2,4 g 4-Dimethylaminopyridin 6 h auf 140°C, versetzt mit Wasser und rührt 1 h bei 60°C, stellt mit Salzsäure auf pH 4 und extrahiert mit Ethylacetat. Man wäscht die organische Lösung mit Kaliumcarbonatlösung, trocknet sie mit Magnesiumsulfat und klärt mit Aktivkohle.Durch Konzentration erhält man 1,0 g (54 %) der Titelverbindung vom Schmp. 164 - 166°C.

50. 3-Formyl-2-methyl-8-(trans-2-morpholinocarbonyloxy-2,3-dihydro-1-indenyloxy)-imidazo[1,2-a]pyridin

a) Zu einer Lösung von 5,0 g 3-Formyl-8-(trans-2-hydroxy-2,3-dihydro-1-indenyloxy)-2-methyl-imidazo[1,2-a]pyridin in 250 ml trockenem Dioxan tropft man bei 20°C 3,7 g Trichlormethylchloroformiat und rührt noch 4 h. Man engt die Suspension im Vakuum zur Trockene ein, verrührt mit Petrolether (40°C) und erhält 7,4 g eines Niederschlags vom Schmp. 111 - 113°C (Zersetzung), der Lösungsmittel enthaltendes 8-(trans-2-chlorocarbonyloxy-2,3-dihydro-1-indenyloxy)-3-formyl-2-methyl-imidazo[1,2-a]pyridin-hydrochlorid darstellt.

b) Man rührt 2,0 g des Niederschlags aus (a), 3 g pulverisiertes wasserfreies Kaliumcarbonat und 0,6 g Morpholin in 40 ml trockenem Dioxan 5 h bei Raumtemperatur. Man tropft 120 ml Wasser zu und kristallisiert den Niederschlag aus Toluol um. Man erhält 1,4 g der Titelverbindung vom Schmp. 200 - 201°C.

51. 3-Formyl-8-[trans-2-(2-hydroxyethylcarbamoyloxy)-2,3-dihydro-1-indenyloxy]-2-methyl-imidazo[1,2-a]-pyridin

Durch analoge Umsetzung wie in Beispiel 50 b mit 2-Aminoethanol statt mit Morpholin erhält man nach Chromatographie an Kieselgel mit Ethylacetat/Isopropylalkohol und Umkristallisieren aus n-Butanol die Titelverbindung vom Schmp. 202 - 204°C.

52. 3-Formyl-8-[trans-2-(N-2-hydroxyethyl-N-methyl-carbamoyloxy)-2,3-dihydro-1-indenyloxy]-2-methyl-imidazo[1,2-a]pyridin

Analog Beispiel 50 b erhält man unter Verwendung von 2-(Methylamino)ethanol anstelle von Morpholin

nach Chromatographie an Kieselgel mit Dichlormethan/Methanol 20:1 in 50 %iger Ausbeute die Titelverbindung vom Schmp. 167 - 168°C (aus Toluol).

53. 8-(trans-2-Carbamoyloxy-2,3-dihydro-1-indenyloxy)-3-formyl-2-methyl-imidazo[1,2-a]pyridin

Analog Beispiel 50 b erhält man durch Einleiten von Ammoniakgas nach Chromatographie an Kieselgel mit Ethylacetat in 72 %iger Ausbeute die Titelverbindung vom Schmp. 238 - 240°C (aus Toluol).

54. 3-Formyl-2-methyl-8-(trans-2-phenylcarbamoyloxy-2,3-dihydro-1-indenyloxy)-imidazo[1,2-a]pyridin

Man rührt 1,0 g 3-Formyl-8-(trans-2-hydroxy-2,3-dihydro-1-indenyloxy)-2-methyl-imidazo[1,2-a]pyridin, 0,42 g Phenylisocyanat, 0,4 g 4-Dimethylaminopyridin und 10 ml Pyridin 12 h bei Raumtemperatur, gießt auf verdünnte Salzsäure, trennt die Verbindung ab und chromatographiert über Kieselgel mit Dichlormethan/Ethylacetat/Cyclohexan (75:15:10). Man erhält in 84 %iger Ausbeute die Titelverbindung vom Schmp. 198 - 199° C (aus Petrolether).
Analog erhält man mit 4-Methoxyphenylisocyanat 3-Formyl-8-[trans-2-(4-methoxyphenylcarbamoyloxy)-2,3-dihydro-1-indenyloxy]-2-methyl-imidazo[1,2-a]pyridin.

55. 8-[trans-2-(2,4-Dimethoxyphenylcarbamoyloxy)-2,3-dihydro-1-indenyloxy]-3-formyl-2-methyl-imidazo[1,2-a]pyridin

Analog Beispiel 54 erhält man mit 2,4-Dimethoxyphenylisocyanat die Titelverbindung vom Schmp. 206 - 208°C (Zersetzung) aus Butanol.

56. 8-(trans-2-tert-Butylcarbamoyloxy-2,3-dihydro-1-indenyloxy)-3-formyl-2-methyl-imidazo[1,2-a]pyridin

1,5 g 3-Formyl-8-(trans-2-hydroxy-2,3-dihydro-1-indenyloxy)-2-methyl-imidazo[1,2-a]pyridin, 1,1 g tert-Butylisocyanat, 1,3 g 4-Dimethylaminopyridin und 10 ml Pyridin werden 24 h bei 50°C gerührt und analog Beispiel 54 aufgearbeitet. Nach Chromatographie an Kieselgel mit Ethylacetat/Isopropylalkohol (9:1) erhält man in 71 %iger Ausbeute die Titelverbindung vom Schmp. 177 - 179°C.

Die nachfolgenden Verbindung 57 und 58 werden in ähnlicher Weise wie in Beispiel 1 beschrieben durch Reduktion entsprechender 3-Formyl-imidazo[1,2-a]pyridine mit Natriumborhydrid hergestellt:

57. 8-(trans-2-Benzyloxy-5-fluor-2,3-dihydro-1-indenyloxy)-3-hydroxymethyl-2-methyl-imidazo[1,2-a]pyridin

Schmp. 188,5 - 189°C (aus Acetonitril).

58. 8-(trans-2-Benzyloxy-5-fluor-2,3-dihydro-1-indenyloxy)-3-formyl-2-methyl-imidazo[1,2-a]pyridin

Durch Umsetzung von 8-(5-Fluor-trans-2-hydroxy-2,3-dihydro-1-indenyloxy)-3-formyl-2-methyl-imidazo-[1,2-a]pyridin mit Benzylbromid in der in Beispiel 48 beschriebenen Weise erhält man die Titelverbindung vom Schmp. 118 - 120°C (aus Isopropanol).

59. 3-Formyl-8-[5-fluor-trans-2-(4-methoxybenzoyloxy)-2,3-dihydro-1-indenyloxy]-2-methyl-imidazo[1,2-a]-pyridin

Analog Beispiel 31 erhält man die Titelverbindung vom Schmp. 186 - 187°C.

60. 3-Hydroxymethyl-8-[5-fluor-trans-2-(4-methoxybenzoyloxy)-2,3-dihydro-1-indenyloxy]-2-methyl-imidazo-[1,2-a]pyridin

Analog Beispiel 2 erhält man die Titelverbindung vom Schmp. 209 - 210°C unter Zersetzung (aus Methanol).

## Ausgangsverbindungen

### A. 3-Formyl-8-(trans-2-hydroxy-2,3-dihydro-1-indenyloxy)-2-methyl-imidazo[1,2-a]pyridin

a) 12 g (68 mmol) 3-Formyl-8-hydroxy-2-methyl-imidazo[1,2-a]pyridin, 18 g (136 mmol) 1,2-Indenoxid und 9,6 ml (68 mmol) Triethylamin werden in 95 ml Methanol/Wasser (4 : 1) 14 h bei 40°C gerührt. Man engt bei 50°C im Rotationsverdampfer ein, versetzt mit 200 ml Eis-Wasser, macht mit Natronlauge alkalisch (ca. pH 13) und filtriert den Niederschlag ab. Das Filtrat wird mit Dichlormethan ausgeschüttelt und die organische Phase auf 250 ml mit Dichlormethan verdünnt, mit dem Niederschlag vereinigt, mit Magnesiumsulfat getrocknet und mit Aktivkohle geklärt. Man versetzt mit 250 ml Toluol, destilliert Dichlormethan ab, klärt in der Siedehitze mit Bleicherde (z.B. Tonsil®), engt auf die Hälfte ein und läßt im Kühlschrank kristallisieren. Man erhält 17,6 g (84 %) der Titelverbindung. Schmp. 200 - 202°C.

b) Alternativ erhält man die Titelverbindung wie folgt:
4,4 g (25 mmol) 3-Formyl-8-hydroxy-2-methyl-imidazo[1,2-a]pyridin, 10,7 g (50 mmol) trans-2-Brom-1-indanol und 13,8 g Kaliumcarbonat werden in 120 ml Methanol/Wasser (4 : 1) 16 h bei 40°C gerührt. Man arbeitet wie unter a beschrieben auf und erhält 4,8 g (62 %) der Titelverbindung. Schmp. 200 - 202°C (aus Methanol).

c) Alternativ erhält man die Titelverbindung wie folgt:
Zu 85,2 g (400 mmol) trans-2-Brom-1-indanol in 600 ml Isopropylalkohol tropft man bei Raumtemperatur unter Rühren 39,7 ml 10 N Kalilauge und rührt noch 15 Min. bei Raumtemperatur. Man destilliert den Isopropylalkohol bei 40°C / 5-8 mbar ab, versetzt mit 350 ml Methanol/Wasser (4 : 1), 30,4 g Kaliumcarbonat und 35,2 g (200 mmol) 3-Formyl-8-hydroxy-2-methyl-imidazo[1,2-a]pyridin und rührt noch 10 h bei 40°C und 8 h bei Raumtemperatur. Man arbeitet wie unter a beschrieben auf und erhält 47,2 g (76,5 %) der Titelverbindung. Schmp. 200 -202°C (aus Methanol).

### B. 8-(5-Fluor-(trans-2-hydroxy-2,3-dihydro-1-indenyloxy)-3-formyl-2-methyl-imidazo[1,2-a]pyridin

Die Titelverbindung vom Schmp. 205°C wird durch Umsetzung von 3-Formyl-8hydroxy-2-methyl-imidazo[1,2-a]pyridin mit 5-Fluor-inden-1,2-oxid nach der in Beispiel A b) angegebenen Arbeitsweise und Umkristallisation aus Methanol erhalten.

### C. 3-Cyanomethyl-8-(trans-2-hydroxy-2,3-dihydro-1-indenyloxy)-2-methyl-imidazo[1,2-a]pyridin

Die Titelverbindung vom Schmp. 181-183°C wird durch Umsetzung von 3-Cyanomethyl-8-hydroxy-2-methyl-imidazo[1,2-a]pyridin mit 1,2-Indenoxid nach der in Beispiel A angegebenen Arbeitsweise erhalten.

## Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgezeichnete Magen- und Darmschutzwirkung bei Warmblütern auf, wobei sie sich durch eine hohe Wirkungsselektivität, das Fehlen wesentlicher Nebenwirkungen und eine große therapeutische Breite auszeichnen. Darüberhinaus können die erfindungsgemäßen Verbindungen zur Behandlung der Osteoporose eingesetzt werden.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Ulcuserkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95% beträgt.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spas molytika, wie z.B. Bietamiverin, Camylofin; Anticholinergica, wie z.B. Oxyphencyclimin, Phencarbamid; Lokalanaesthetika, wie z.B. Tetracain, Procain; Antibiotika, wie Penicilline, Tetracycline etc. gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise $H_2$-Blockern (z.B. Cimetidin, Ranitidin), mit sogenannten peripheren Anticholinergika (z.B. Pirenzepin, Telenzepin, Zolenzepin) mit dem Ziel, die Hauptwirkung in additivem oder überadditivem Sinn zu verstärken und/oder die Nebenwirkungen zu eliminieren oder zu verringern, oder ferner mit antibakteriell wirksamen Substanzen (wie z.B. Cephalosporinen, Tetracyclinen, Nalidixinsäure, Penicillinen etc.) zur Bekämpfung von Campylobacter pyloridis.

## Pharmakologie

Die ausgezeichnete Magenschutzwirkung und die magensekretionshemmende Wirkung der erfindungsgemäßen Verbindungen kann in Untersuchungen an tierexperimentellen Modellen nachgewiesen werden. Die in der nachstehend aufgeführten Tabelle untersuchten erfindungsgemäßen Verbindungen sind mit

Nummern versehen worden, die den Nummern der Beispiele entsprechen.

Prüfung der sekretionshemmenden Wirkung am perfundierten Rattenmagen

In der folgenden Tabelle 2 ist der Einfluß der erfindungsgemäßen Verbindungen nach intravenöser Gabe auf die durch Pentagastrin stimulierte Säuresekretion des perfundierten Rattenmagens in vivo dargestellt.

Tabelle 2

| Lfd. Nr. | N (Anzahl der Tiere) | Dosisbereich (µmol/kg) i.v. | Maximale Hemmung der Säureausscheidung | |
|---|---|---|---|---|
| | | | (%) | ca. ED50 +) (µmol/kg) i.v. |
| 2 | 15 | 1,0 - 3,0 | 48 - 94 | 1,1 |
| 5 | 18 | 0,6 - 2,0 | 18 - 98 | 0,8 |

+) ED50 = Dosis (interpoliert), die eine maximale Hemmung der HCl-Sekretion um 50 % bewirkt.

Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Ösophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca.50-100 ml) wurde der Magen mit 37°C warmer physiologischer NaCl-Lösung kontinuierlich durchströmt (0,5 ml/min, pH 6,8-6,9; Braun-Unita I). In dem jeweils im 15 min-Abstand aufgefangenen (25 ml Meßzylinder) Effluat wurde der pH-Wert (pH-Meter 632, Glaselektrode EA 147; ⌀ = 5 mm, Metrohm) sowie durch Titration mit einer frisch zubereiteten 0,01 N NaOH bis pH 7 (Dosimat 655 Metrohm) die sezernierte HCl bestimmt.

Die Stimulation der Magensekretion erfolgte durch Dauerinfusion von 1µg/kg (=1,65 ml/h) i.v. Pentagastrin (V. fem. sin.) ca 30 min nach Operationsende (d.h. nach Bestimmung von 2 Vorfraktionen). Die zu prüfenden Substanzen wurden intravenös (V. jugularis sin.) in 1 ml/kg Flüssigkeitsvolumen 60 min nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 37,8 - 38°C gehalten.

Als Maß für die sekretionshemmende Wirkung diente die maximale Abnahme der Säureausscheidung (15 min-Fraktionen) jeder behandelten Gruppe gegenüber derjenigen der unbehandelten Kontrollgruppe (=100 %). Die ED50 bezeichnet diejenige Dosis, die eine maximale Hemmung der HCl-Sekretion um 50 % bewirkt.

**Ansprüche**

1. Imidazopyridine der Formel I

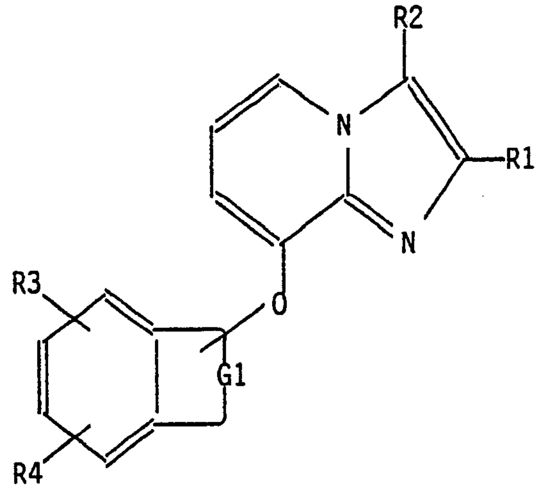

(I)

worin
R1 Wasserstoff (H), 1-4C-Alkyl, Hydroxy-1-4C-alkyl, Halo-1-4C-alkyl, Cyano-1-4C-alkyl oder 1-4C-Alkoxycarbonyl,
R2 Wasserstoff (H), 1-4C-Alkyl, Formyl, Hydroxy-1-4C-alkyl, Halo-1-4C-alkyl, Cyano-1-4C-alkyl oder Di-1-4C-alkylamino-1-4C-alkyl,
R3 Wasserstoff (H), 1-4C-Alkoxy oder Halogen,
R4 Wasserstoff (H), 1-4C-Alkoxy oder Halogen und
G1 eine substituierte Gruppe der Formel $-CH(OR5)-CH_2-CH_2-$, $-CH_2-CH(OR5)-CH(CH_3)-$, $-CH_2$ $-CH(OR5)-CH_2-$,$-CH(OR5)-CH_2-CH_2-CH_2-$, $-CH_2-CH(OR5)-CH_2-CH_2-$, $-CH(OR5)-CH_2-CH_2-CH_2-CH_2-$ oder $-CH_2-CH(OR5)-CH_2-CH_2-CH_2-$ bedeutet in der
R5 1-6C-Alkyl, 3-7C-Cycloalkyl, 1-4C-Alkoxy-1-4C-alkyl, Aryl-1-4C-alkyl, 1-6C-Alkylcarbonyl, 3-7C-Cycloalkylcarbonyl, Aryl-1-4C-alkylcarbonyl, 1-4C-Alkoxy--4C-alkylcarbonyl, 1-4C-Alkoxy-1-4C-alkoxy-1-4C-alkylcarbonyl, 1-4C-Alkoxycarbonyl, 1-4C-Alkoxy-1-4C-alkoxycarbonyl, Arylcarbonyl, Furoyl oder eine Carbamoylgruppe der Formel

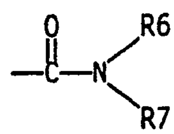

bedeutet, worin
R6 Wasserstoff (H), 1-4C-Alkyl, Hydroxy-1-4C-alkyl oder gemeinsam mit R7 und unter Einschluß des Stickstoffatoms, an das beide gebunden sind, Pyrrolidino, Piperidino, Tetrahydroazepino, Piperazino, N-Methylpiperazino oder Morpholino bedeutet und
R7 Wasserstoff. (H), 1-4C-Alkyl, Aryl oder gemeinsam mit R6 und unter Einschluß des Stickstoffatoms, an das beide gebunden sind, Pyrrolidino, Piperidino, Tetrahydroazepino, Piperazino, N-Methylpiperazino oder Morpholino bedeutet, wobei
Aryl Phenyl oder substituiertes Phenyl mit einem, zwei oder drei Substituenten aus der Gruppe Halogen, 1-4C-Alkyl oder 1-4C-Alkoxy bedeutet,
und ihre Salze.

2. Verbindungen der Formel I nach Anspruch 1, worin
R1 1-4C-Alkyl,
R2 1-4C-Alkyl, Formyl, Hydroxymethyl oder Cyanomethyl,
R3 Wasserstoff oder Halogen,
R4 Wasserstoff und
G1 eine substituierte Gruppe der Formel $-CH_2-CH(OR5)-CH_2-$ bedeutet, in der
R5 1-4C-Alkyl, 1-4C-Alkoxy-1-4C-alkyl, Aryl-1-4C-alkyl, 1-4C-Alkylcarbonyl, 3-7C-Cycloalkylcarbonyl, Aryl-1-4C-alkylcarbonyl, 1-4C-Alkoxy-1-4C-alkylcarbonyl, 1-4C-Alkoxy-1-4C-alkoxy-1-4C-alkylcarbonyl, 1-4C-Alkoxycarbonyl, Arylcarbonyl, Furoyl oder eine Carbamoylgruppe der Formel

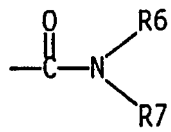

bedeutet, worin
R6 Wasserstoff (H), 1-4C-Alkyl, Hydroxy-1-4C-alkyl oder gemeinsam mit R7 und unter Einschluß des Stickstoffatoms, an das beide gebunden sind, Morpholino bedeutet und
R7 Wasserstoff (H), 1-4C-Alkyl, Aryl oder gemeinsam mit R6 und unter Einschluß des Stickstoffatoms an das beide gebunden sind, Morpholino bedeutet, wobei
Aryl Phenyl oder substituiertes Phenyl mit einer, zwei oder drei 1-4C-Alkoxygruppen bedeutet,
und ihre Salze.

3. Verbindungen nach Anspruch 2, gekennzeichnet durch die Formel I*

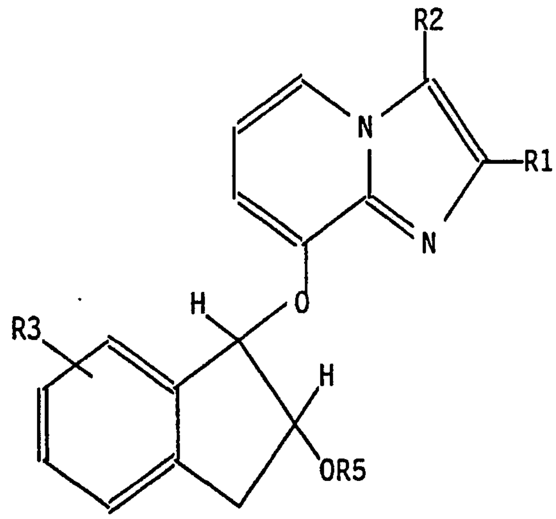

(I*)

worin R1, R2, R3 und R5 die in Anspruch 2 angegebenen Bedeutungen haben, und ihre Salze.

4. Verbindungen nach Anspruch 2, gekennzeichnet durch die Formel Ia

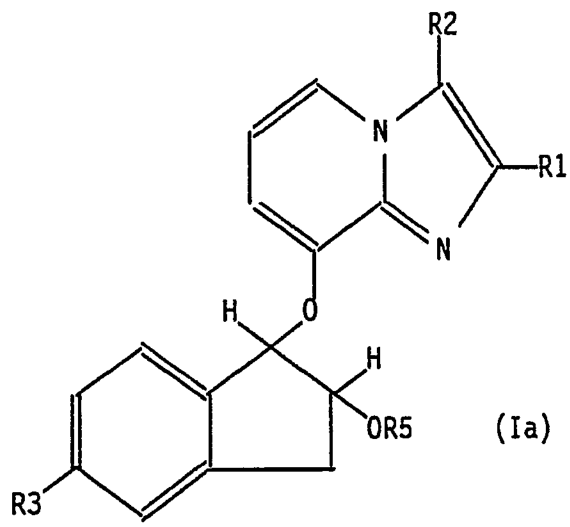

(Ia)

worin
R1 1-4C-Alkyl bedeutet,
R2 1-4C-Alkyl, Hydroxymethyl oder Cyanomethyl,
R3 Wasserstoff oder Halogen bedeutet und

R5 die in Anspruch 2 angegebenen Bedeutungen hat, und ihre Salze.

5. Verbindungen nach Anspruch 2, gekennzeichnet durch die Formel Ia

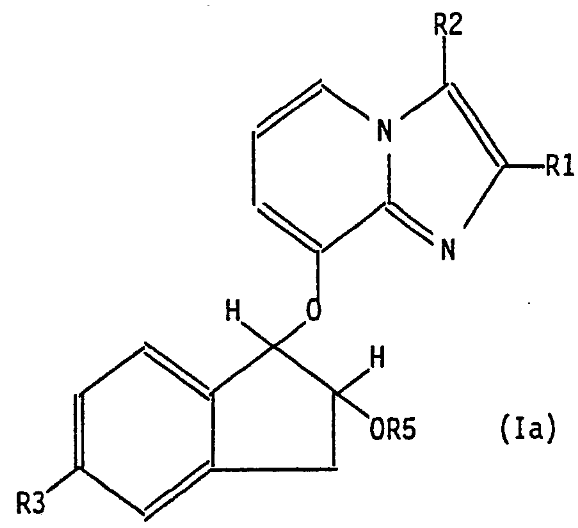

(Ia)

worin
R1 1-4C-Alkyl bedeutet,
R2 Hydroxymethyl oder Cyanomethyl,
R3 Wasserstoff oder Halogen bedeutet und
R5 die in Anspruch 2 angegebenen Bedeutungen hat,
und ihre Salze.

6. Verfahren zur Herstellung der Verbindungen der Formel I nach Anspruch 1 und ihrer Salze, dadurch gekennzeichnet, daß man in Verbindungen der Formel I, worin R1, R2, R3, R4 und G1 die in Anspruch 1 angegebenen Bedeutungen haben und R5 Wasserstoff bedeutet, das Wasserstoffatom R5 durch einen Rest R5, der die in Anspruch 1 angegebenen Bedeutungen hat, substituiert und gewünschtenfalls anschließend erhaltene Verbindungen, worin R2 die Bedeutung Formyl hat, zu den Verbindungen der Formel I, worin R2 die Bedeutung Hydroxymethyl hat, reduziert, und/oder gewünschtenfalls anschließend erhaltene Salze in die freien Verbindungen oder erhaltene freie Verbindungen in die Salze überführt, wobei die Ausgangsverbindungen I als solche oder in Form ihrer Salze eingesetzt werden.

7. Verfahren zur Herstellung der Verbindungen der Formel I nach Anspruch 1, worin R2 Hydroxymethyl bedeutet und R1, R3, R4 und G1 die in Anspruch 1 angegebenen Bedeutungen haben, und ihrer Salze, dadurch gekennzeichnet, daß man in Verbindungen der Formel I, worin R2 Formyl bedeutet, R5 Wasserstoff bedeutet und R1, R3, R4 und G1 die in Anspruch 1 angegebenen Bedeutungen haben, das Wasserstoffatom R5 durch einen Rest R5, der die in Anspruch 1 angegebenen Bedeutungen hat, substituiert und anschließend erhaltene Verbindungen reduziert, und gewünschtenfalls anschließend erhaltene Salze in die freien Verbindungen oder erhaltene freie Verbindungen in die Salze überführt, wobei die Ausgangsverbindungen I als solche oder in Form ihrer Salze eingesetzt werden.

8. Arzneimittel enthaltend eine oder mehrere Verbindungen nach einem oder mehreren der Ansprüche 1 bis 5 und/oder ihre pharmakologisch verträglichen Salze.

9. Verbindungen nach einem oder mehreren der Ansprüche 1 bis 5 und ihre pharmakologisch verträglichen Salze zur Anwendung bei der Verhütung und Behandlung gastrointestinaler Krankheiten.

10. Verwendung von Verbindungen nach einem oder mehreren der Ansprüche 1 bis 5 und ihren pharmakologisch verträglichen Salzen zur Herstellung von Arzneimitteln für Verhütung und Behandlung gastrointestinaler Krankheiten.

Europäisches Patentamt

# EUROPÄISCHER RECHERCHENBERICHT

Nummer der Anmeldung

EP 89 12 0355

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 204 285 (FUJISAWA)<br>* Ansprüche 1,21; Beispiel 12 *<br>--- | 1,8 | C 07 D 471/04<br>A 61 K 31/435//<br>(C 07 D 471/04<br>C 07 D 235:00<br>C 07 D 221:00 ) |
| D,A | EP-A-0 033 094 (SCHERING)<br>* Ansprüche 1,8; Seite 73 *<br>--- | 1,8 | |
| P,A | EP-A-0 290 003 (BYK)<br>* Ansprüche 1,10 *<br>--- | 1,8 | |
| P,A | EP-A-0 299 470 (BYK)<br>* Ansprüche 1,11 *<br>----- | 1,8 | |

RECHERCHIERTE SACHGEBIETE (Int. Cl.5)

C 07 D 471/00
A 61 K 31/00

Der vorliegende Recherchenbericht wurde für alle Patentansprüche erstellt

| Recherchenort | Abschlußdatum der Recherche | Prüfer |
|---|---|---|
| DEN HAAG | 08-02-1990 | ALFARO I. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)